# EUROPEAN PATENT SPECIFICATION

(11) **EP 4 588 429 B1**
(45) Date of publication and mention of the grant of the patent: **25.02.2026**
(21) Application number: 24461511.8
(22) Date of filing: 17.01.2024
(51) Int. Cl.: A61B 5/00, G06T 7/11

(54) **A COMPUTER IMPLEMENTED METHOD, COMPUTER PROGRAM PRODUCT AND COMPUTER SYSTEM FOR ENHANCING DIAGNOSIS**
COMPUTERIMPLEMENTIERTES VERFAHREN, COMPUTERPROGRAMMPRODUKT UND COMPUTERSYSTEM ZUR VERBESSERUNG DER DIAGNOSE
PROCÉDÉ MIS EN UVRE PAR ORDINATEUR, PRODUIT DE PROGRAMME INFORMATIQUE ET SYSTÈME INFORMATIQUE POUR AMÉLIORER LE DIAGNOSTIC

(43) Date of publication of application: 23.07.2025
(73) Proprietor: HEMOLENS DIAGNOSTICS SPOLKA Z OGRANICZONA ODPOWIEDZIALNOSCIA, 54-202 Wroclaw (PL)
(72) Inventor: Zamorski, Maciej, 50-231 Wroclaw (PL); Gajowczyk, Milosz, 48-200 Prudnik (PL); Konopczynski, Tomasz, 54-105 Wroclaw (PL)
(74) Representative: Bury & Bury

(56) References cited:
- EP-A1- 4 069 076
- WO-A1-2021/111474
- WANG ZIYANG ET AL: "Densely Connected Swin-UNet for Multiscale Information Aggregation in Medical Image Segmentation", 2023 IEEE INTERNATIONAL CONFERENCE ON IMAGE PROCESSING (ICIP), IEEE, 8 October 2023 (2023-10-08), pages 940 - 944, XP034466873, [retrieved on 20230911], DOI: 10.1109/ICIP49359.2023.10222451
- SIMON DAHAN ET AL: "Surface Vision Transformers: Flexible Attention-Based Modelling of Biomedical Surfaces", ARXIV.ORG, CORNELL UNIVERSITY LIBRARY, 201 OLIN LIBRARY CORNELL UNIVERSITY ITHACA, NY 14853, 7 April 2022 (2022-04-07), XP091201198
- KONG FANWEI ET AL: "Learning Whole Heart Mesh Generation From Patient Images for Computational Simulations", IEEE TRANSACTIONS ON MEDICAL IMAGING, IEEE, USA, vol. 42, no. 2, 2 November 2022 (2022-11-02), pages 533 - 545, XP011933656, ISSN: 0278-0062, [retrieved on 20230202], DOI: 10.1109/TMI.2022.3219284
- UDARANGA WICKRAMASINGHE ET AL: "Voxel2Mesh: 3D Mesh Model Generation from Volumetric Data", ARXIV.ORG, CORNELL UNIVERSITY LIBRARY, 201 OLIN LIBRARY CORNELL UNIVERSITY ITHACA, NY 14853, 8 December 2019 (2019-12-08), XP081633488

## Description

### FIELD OF THE INVENTION

A computer implemented method, computer program product and computer system for enhancing diagnosis by processing medical images of organs and providing segmentation thereof.

### STATE OF THE ART

Diagnosis of heart and vascular diseases requires analysis of images of heart. Representation of such images is usually done according to one of the standardized cardiac scales such as AHA 17 - American Heart Association 17-segment model of LV myocardium in which a heart image is oriented and divided into 17 segments to enhance analysis and diagnosis by a physician. AHA 17 is described in Cerqueira MD, Weissman NJ, Dilsizian V, Jacobs AK, Kaul S, Laskey WK, Pennell DJ, Rumberger JA, Ryan T, Verani MS; American Heart Association Writing Group on Myocardial Segmentation and Registration for Cardiac Imaging. Standardized myocardial segmentation and nomenclature for tomographic imaging of the heart. A statement for healthcare professionals from the Cardiac Imaging Committee of the Council on Clinical Cardiology of the American Heart Association. Circulation. 2002 Jan 29;105(4):539-42. doi: 10.1161/hc0402.102975. PMID: 11815441.

EP4069076 discloses a method of processing a cardiac image or a myocardium model for displaying a cardiac scale to the steps of pre-processing, transforming and orientation, combining multiple images, demarcating segment boundaries, superimposing with additional estimated set of parameters in particular segments and displaying. It would be generally desirable to provide physician for more parameters for diagnosis fused with cardiac images. Attempts are made for a use of computational fluid dynamics (CFD) simulations with model obtained in the same process in which medical image is segmented into the cardiac scale.

It is noted that term segmentation in the art is used in two meanings relevant for the present patent application. The first meaning of segmentation relates to dividing image of a heart or model of a heart into segments according to cardiac scale. This meaning is going to be referred to as medical segmentation. But, when it concerns the heart: cardiac segmentation. The second meaning of the segmentation relates to fully or partly automated, and often involving an artificial intelligence, process of creating the model of object or organ using its representation in a three dimensional image e.g. but not only for purpose of CFD. For the sake of clarity in the present document the process of creating a model from the three dimensional image will be referred to as semantic segmentation.

US8734357 discloses obtaining patients medical imaging data of a heart, semantic segmentation to obtain 3D model of myocardial tissue and simulating perfusion using the model of myocardium and additional models of arteries.

Obtaining a consistent model applicable for both cardiac segmentation according to a cardiac scale and for semantic segmentation to be used in CFD simulation, appears to be non-trivial task. On the other hand it is highly desirable for the physician to have consistent data.

Machine learning is playing an increasingly important role in imaging and the diagnostics of cardiovascular diseases. For example, machine learning techniques such as deep learning have been found to be highly suitable for creating anatomical models from medical images, e.g. for the purpose of classification and semantic segmentation of image content in medical image processing of vascular system. Although machine learning method proved to be useful in creating organ models to be visualized according to cardiac scale and creating models for CFD simulations - it is noted that obtaining a model that could be used for both applications is not reported in the art despite obvious advantages of showing to physician the same model which has been used for simulation allowing to obtain certain parameters.

It is well known, that machine learning algorithms are trained using training data as input. After such training, a trained machine learning algorithm can be applied to new data, e.g. to create a model from medical image of an anatomical structure in a process referred to as semantic segmentation.

EP3570249 discloses machine learning in an analysis of images of a myocardium. US10896508B2 discloses a method which comprises collecting a set of chest computed tomography angiography (CTA) images scanned in the axial view and a manual creation of models from the images, for each one of multiple organs. The images are subjected to preprocessing such that they share the same field of view (FOV). Thereafter, both the images and models are used in a supervised training of a deep neural network for creating models from images. The loss function is determined from a multi-dice score that is the summation of the dice scores for all the multiple organs, each dice score being computed as the similarity between the model obtained manually, and the output of the network for one of the organs. Input pre-processed image is then analyzed on the trained network, thereby obtaining a model from the given image. Consequently the output model is then subjected to smoothing.

CN117058163 indicates advantages of use of swin transformers in machine learning assisted creation of models using the medical images including a myocardium. Swin transformers relate to the dominant sequence transduction models are based on complex recurrent or convolutional neural networks in an encoder-decoder configuration.

Hatamizadeh, Ali, et al. "Swin unetr: Swin transformers for semantic segmentation of brain tumors in mri images." International MICCAI Brainlesion Workshop. Cham: Springer International Publishing, 2021 teaches that Swin transformers relate to Fully Convolutional Neural Networks (FCNNs). In recent years, FCNN approaches have become the de facto standard for 3D medical image processing. Unet network is an architecture of neural network for image segmentation known since 2015 (Olaf Ronneberger, Philipp Fischer, and Thomas Brox. U-net: Convolutional networks for biomedical image segmentation. In International Conference on Medical image computing and computer-assisted intervention, pages 234-241. Springer, 2015) using encoder to reduce dimension of the problem and then decoder to produce segmentation map. Transformers are efficient approach to implement the encoder. Swin approach known from article by Ze Liu, Yutong Lin, Yue Cao, Han Hu, Yixuan Wei, Zheng Zhang, Stephen Lin, Baining Guo, Swin Transformer: Hierarchical Vision Transformer using Shifted Windows, https://doi.org/10.48550/arXiv.2103.14030 is applied to transformer to increase precision. Generally term vision transformers is used to describe transformer methods for image processing and segmentation - Swin transformer are particular kind thereof. Vision transformers appeared to be very efficient in semantic segmentation. However, state of the art equipment is still not fast and affordable enough for processing whole 3D images of larger sizes with vision transformers.

Swin UNEt TRansformers (Swin UNETR) is a model introduced to mitigate the problem mentioned above. Specifically, the task of 3D semantic creation of model from 3D image is reformulated as a sequence to sequence prediction problem wherein multi-modal input data is projected into a 1D sequence of embedding and used as an input to a hierarchical Swin transformer as the encoder.

Computer implemented segmentation techniques are applied to myocardium to provide assessment according to cardiac scale such as AHA 17 segments (US20220414865, US10964120) or for CFD simulations (US8831320, EP2932469, US8311747). Designing segmentation algorithm that provides a model applicable for both using assessments based on cardiac scale and for CFD simulations is difficult. Machine learning using transformers, especially swin transformer, provides generally good accuracy expected in reproducing general shape desired in cardiac scale assessment. Although not very popular in this field, yet are mentioned among algorithms used for processing images of living organisms, eg. from EP4264611. However, it is very difficult to train neural network using transformer to make it insusceptible to errors due to anomalies.

In the medical images parts of myocardium subjected to ischemia are very similar to surrounding tissue and is easily confused therewith. Additionally, myocardium changes its shape during a normal cardiac cycle. Consequently, most accurate models are not physically adequate for simulation, because they are not always "tight" as misclassification of myocardial tissue subjected to ischemia, which may occasionally cause artificial "holes" in the model. It is however beneficial to provide image a person performing diagnosis with cardiac scale segments such as AHA consistent with model used for CFD simulation and provide CFD estimated parameters in particular segments.

EP1966756 discloses alternative approach for creating model from 3D medical image based on starting with mesh of predefined shape and deforming it to match medical image. Mesh deforming algorithms, however are less accurate and result in larger and thicker models difficult to use in simulations. Example of this approach is disclosed in detail in Kong et al. MeshDeformNet (https://arxiv.org/abs/2102.07899).

### PROBLEMS TO BE SOLVED

Medical images are often difficult to read even for trained physicians, that is why medical segmentation according to various scales are applied and simplified models of organs are created. These models tend often to be inadequate due to certain artifacts. Presenting inadequate model to the physician may affect diagnosis. This is a particular problem, when it is advisable to use the model in simulation to provide additional parameters, which may be affected by inadequate model.

Combination of both model applicable for simulation and adequate for showing to the physician in a manner easy to read proved to be very difficult to obtain.

In particular ischemic myocardium tissues are in certain situations represented in medical images in a very similar manner to the surrounding tissue. Therefore machine learning methods for semantic segmentation to create 3D models from three dimensional images based on semantic context analysis are difficult to be trained adequately. Moreover are susceptible to occasional failures making the results inadequate for CFD simulation. Typical failure is related to non-existing opening in a model of myocardium being a result of semantic segmentation. Other methods are often subjected to even greater errors or occasionally gross errors causing misshaping the model entirely.

### SUMMARY OF THE INVENTION

A computer implemented method for enhancing diagnosis by processing medical images according to the invention comprises: a step of receiving a medical image of an organ comprising three dimensional representation thereof, a step of preprocessing the medical image, a step of semantic segmentation of the medical image to obtain a model of the organ, a step of orientation of the model, a step of medical segmentation of the model of the organ a step of preparing visualization of the model of the organ and/or the medical image together with the result of medical segmentation. According to the invention the model of the organ is an ensembled model. The step of semantic segmentation comprises: applying a vision transformer to obtain a first model of myocardium, applying a mesh deformer to obtain a second model of myocardium, and ensembling of the first model and the second model to the obtain ensembled model. The inventors have realized, that models obtained with vision transformers and with mesh deformers are in many ways complementary and ensembling thereof can provide universal models that can be shown together with results of medical segmentation for diagnosis purpose and simultaneously used for various medical simulations - particularly for CFD simulations. It has been noted that vision transformers provide generally more accurate models, but are more prone to local failures. Models resulting from mesh deformation are less fine, but also less susceptible to local failures.

Advantageously the computer implemented method for enhancing diagnosis of cardiovascular diseases by processing medical images, comprises a step of receiving a medical image of myocardium comprising three dimensional representation thereof. The method further comprises: a step of preprocessing the medical image, a step of semantic segmentation of the medical image to obtain a model of myocardium, a step of orientation of the model, and a step of medical segmentation the model of myocardium according to a cardiac scale and a step of preparing visualization of the model of myocardium and/or the medical image together with the cardiac scale. According to the invention the model of myocardium is an ensembled model. The step of creating the model of the myocardium from the medical image comprises applying a vision transformer to obtain a first model of myocardium, applying a mesh deformer to obtain a second model of myocardium and ensembling of the first model and the second model to the obtain ensembled model. The method further comprises a step of computational fluid dynamics simulation comprising receiving patient specific parameters for formulation boundary conditions and running the computational fluid dynamics simulation with boundary conditions to determine an additional parameter. The additional parameter is included in the visualization prepared in the step. The result of ensembling the first and the second model proved to be adequate to show to the physician and to use with computational fluid dynamics simulations.

Advantageously the medical image is computed tomography angiography image. CTA images are specifically adapted to analysis of blood guiding organs.

The cardiac scale advantageously is 17 AHA scale.

The ensembling, advantageously comprises using models obtained from mesh deformer to repair faulty parts of models obtained with vision transformer. Alternatively models obtained with mesh deformer are used to replace first models from vision transformer in case when fault of the first model is detected. The former approach is particularly useful as less accurate parts of the second models can be used to repair local errors in generally more accurate first model.

The additional parameter determined in the step of computational fluid dynamics simulation is selected from a group comprising myocardial blood flow (MBF), time to peak (TTP), tissue transit time (TTT) and myocardial blood volume (MBV).

The step of computational fluid dynamics simulation is advantageously used to determine two or more additional parameters which are then presented in the step of visualization.

The ensembling advantageously comprises analysis of the first model obtained with vision transformer in subsequent slices, wherein every slice is analyzed with respect to topological homeomorphism to a set of predefined structures and slice that fails to have topological homeomorphism to expected shape is corrected with corresponding slice from the second model obtained with mesh deformer.

Advantageously the ensembling step comprises postprocessing of the ensembled model.

Computer program product according to the invention, comprises instructions which, when the program is executed by a computer, cause the computer to carry out the method according to the invention.

A computer system according to the invention comprises terminal and server. The terminal is adapted to upload data to the server and the server is adapted to process the data and return the result while the terminal is further adapted to visualize the result. The server is adapted to realize the method according to the invention and deliver the prepared to visualization to the terminal. The terminal is adapted to show the visualization.

### DESCRIPTION OF DRAWINGS

The invention has been described below in detail, with reference to the following figures, wherein Fig. 1 shows a flow chart of a method according to the invention, Fig. 2a shows a training model of myocardium with four regions of points, Fig. 2b shows an expected topology of a slice of a model of myocardium corresponding to cross-section in the middle thereof, Fig. 2c shows a slice at the bottom of the myocardium, Fig. 2d shows a faulty slice or a slice in the top part of the myocardium, and Fig. 3 shows a flow chart of an example of method of ensembling two models, Fig. 4 shows a model of myocardium with segmentation according to AHA 17 scale with an intensity representing the MBF (myocardial blood flow) .

### EMBODIMENTS OF THE INVENTION

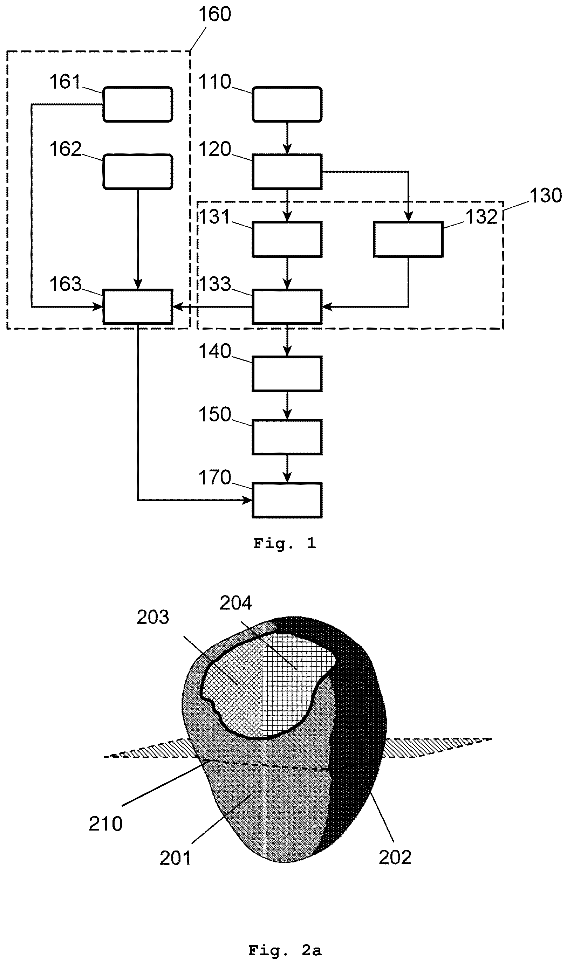

The computer implemented according to embodiment of the invention is described below with reference to Fig. 1. The method comprises a step of receiving one or more medical images of myocardium comprising three dimensional representation thereof.

Computed tomography angiography (CTA) imaging is a technique dedicated for analysis of arteries and veins and following the blood flow. Present embodiment refers to processing medical image being a CTA image obtained in Cardiac Computed Tomography Angiography (CCTA) in any of the formats DICOM / Nifti / NRRD. The CTA image is received in a step of receiving medical image **110.** In the present embodiment step of receiving CTA image consists in loading the images from the disk. Nevertheless, this step may comprise also receiving image via computer network or taking it with dedicated equipment.

The method according to the invention uses also additional physical parameters of patient received in the step **161** and models of organs complementary to myocardium received in step **162.** Physical parameters and models of complementary organs are later used for extraction of additional parameter in CFD simulation - step **163.** The CFD can be also performed without models of additional organs but this requires broader range of physical parameters allowing to formulate adequate boundary condition - as disclosed in EP4002388.

CTA image received in the step **110** is thereafter subjected to preprocessing in the step **120.** In the present embodiment the preprocessing step **120** comprises scaling and normalization of dimensions as well as scaling and normalization of the values to predefined dynamic range expressed in Hounsfield units. Preferred dynamic range is -300 to 1100 HU which allows proper observation of myocardium. Additionally parts non-relevant parts of image are removed to enable more efficient processing with the neural network. In the present embodiment voxel size is scaled to 0.8 mm, however different sizes can be applied. Generally scope from 0.3 to 1.5 mm worked quite well, to provide accurate result fast.

Preprocessed CTA image is thereafter subjected to segmentation in the step **130.** The segmentation comprises use of two diametrically different algorithms applied in two steps **131, 132.**

In the step of transformer segmentation **131** the preprocessed CTA image is subjected to machine learning segmentation using stacked fully connected layers algorithm. In the present embodiment swin transformer is used, specifically the algorithm disclosed in Hatamizadeh, Ali, et al. "Swin unetr: Swin transformers for semantic segmentation of brain tumors in mri images." International MICCAI Brainlesion Workshop. Cham: Springer International Publishing, 2021 (https://arxiv.org/pdf/2201.01266.pdf). Swin transformer segmentation provides the first model of the myocardium.

In the step of mesh deformer segmentation **132** second model is created using algorithm described by Kong et al. MeshDeformNet (https://arxiv.org/abs/2102.07899). The fundamental idea behind this method is to directly predict meshes from volumetric data in order to overcome the shortcomings, such as disconnected regions and inaccuracies in surface topology. This is accomplished by introducing to the standard UNet-like architecture an additional mesh deformation module acting as a decoder. The graph decoder, taking a spherical mesh as the default input, employs graph convolutions to deform the prior mesh model into the desired output, while the standard encoder serves as regularization.

The MeshDeformNet architecture is well known in the art and available publicly in library https://github.com/fkong7/MeshDeformNet. In the simplest variant MeshDeformNet, for the purpose of model training uses various loss functions over a surface defined by a mesh. The mesh is defined with points nodes connected with edges. One of loss functions is the symmetrical chamfer loss. Chamfer loss measures the sum of minimal distances from every point in a first group of points defining a surface generated and processed by the neural network to the points in the second group of points being a ground truth as well as from every point in the second group to points in the first group. This approach is applicable in the present invention. However, it can be further improved as explained below. Myocardium is relatively dissimilar to sphere. It has a concave part. As shown in Fig. 2a the myocardium has outer wall **201, 202** and also inner wall **203, 204.**

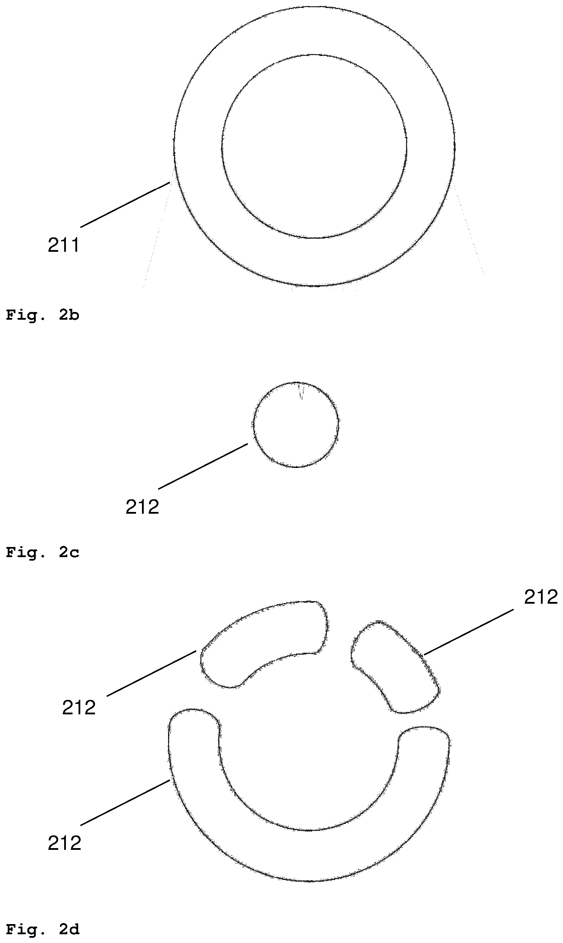

Fig. 2a further shows a slice 210 taken at cross-section through the model shown as plane in Fig. 2. In the middle part of the model slices **210** have topology **211** shown in Fig. 2b. In the bottom part of the model slices have topology **212** shown in Fig. 2c. Slices in the top portion of the model have different shape but the same topology - as shown in Fig. 2d. Shape shown in fig 2d indicates clearly an error, if is observed in the middle of the model. This kinds of errors are specific for models obtained with vision transformer - caused by low difference between ischemia in myocardium tissue and surrounding tissues.

It became apparent, that due to specific shape of myocardium, using symmetrical chamfer loss reduces the ability of the neural network to deform the prior, convex mesh (sphere) into expected shape of myocardium, which is non-convex. The neural network tends to be trained to prefer deformations including moving only small fraction of points into inner wall of produced myocardial model, resulting in significant drop of segmentation fidelity in terms of metrics such as Chamfer Distance.

To address this shortcoming, and improve distribution of points an extension to the original chamfer loss calculations was introduced. To represent destination shape better, the original spherical prior shape was divided into four parts evenly sized in terms of contained points. The training myocardium-shape outputs were divided in similar fashion, so that four respective regions **(201, 202, 203, 204)** matched corresponding parts of points of the shape. Two regions **(201, 202)** comprised the outer points of the myocardium and the other two **(203, 204)** the inner points of the myocardium.

A simple and working approach to divide points of initial sphere (starting shape of MeshDefromNet) into adequate groups, is to split the surface of the sphere into upper and lower hemisphere and then split each of the hemispheres by half. Then the sphere divided into regions is rotated according to orientation of the myocardium so that plane of division into hemispheres is perpendicular to longitudinal axis of the myocardium.

Division of points into four sets corresponding to four regions resulted in more granular comparison and thus enabled training of the neural network enabling deformation of the input shape resulting in output shape, that better corresponds to actual shape of myocardium. Thus an issue of uneven number of points per part of the output and provided a smoother semantic segmentation as a result.

Typically MeshDeformNet returns two outputs volumetric and mesh. The mesh output is by definition always fluid tight which makes it useful for CFD simulations. Still the method is susceptible to random failures and generally is less precise than vision transformers and tends to output models being too thick. Models obtained entirely with MeshDeformNet are difficult to be used in simulation.

In the step **133,** the first myocardium model resulting from semantic segmentation with vision transformer [UNetr] available publicly in library https://docs.monai.io/en/stable/_modules/monai/networks/nets/ swin_unetr.html ) applied in the step **131** and the second model resulting from semantic segmentation with mesh deformer [MeshDeformNet] applied in the step **132** are fused together in the ensembling process. There are multiple ways to carry on the ensembling of the first and the second model. A simplest direction would be to detect fault in the first model and replace it with the second model as the second models obtained with mesh deformers are less accurate but more error proof than the models obtained with vision transformers. More elaborate way approach involves using only part of the second model to correct faulty part of the first model.

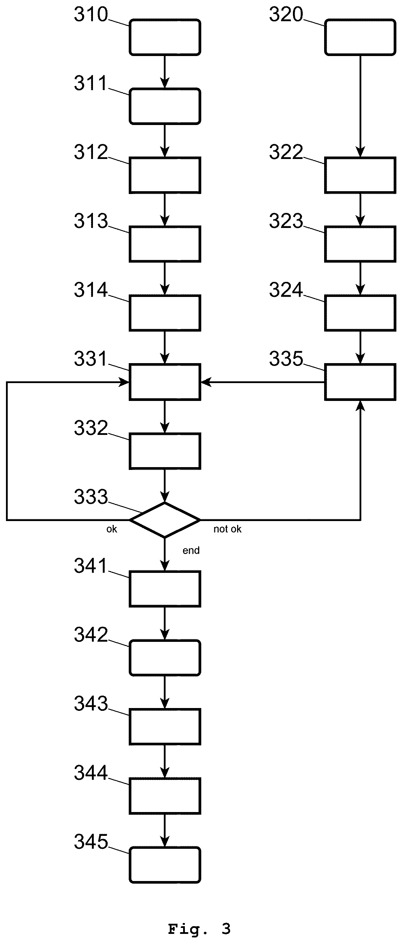

In the present embodiment, the ensembling is realized as described below with reference to Fig. 3. In the step **320** the second model, specifically the mesh output of MeshDeformNet **132** is received and voxelized to obtain volumetric representation of the second model. Then characteristic points of the model are detected in the step **322** and subsequently the model is re-oriented in step **323** and divided into slices **210** in the step **324.** The characteristic points include a mitral valve, an apex, and a septum.

The first model obtained in step **131** with vision transformer [UNetr] is received in the step **310.** Then the first model is step **311** undergoes morphological closing to prevent small holes in produced segmentations.

Next, in step **312,** characteristic points of a myocardium are detected in the first model. The characteristic points include a mitral valve, an apex, and a septum. Then in the step **313** the first model is re-oriented according to the positions of the characteristic points. Re-orientation is done automatically and with rotation and shift being calculated based on the characteristic points. First, the longitudinal axis for each model is established, based on the centers of the mitral valve and apex points. The horizontal rotation is then determined, as an angle between the z-axis and the longitudinal axis, in order to align the myocardium vertically, i.e., in such a way, that apex becomes the bottom point of the model. Subsequently, the supplementary semi-axis is established as orthogonal to the longitudinal axis and passing through the septum point. The angle between the supplementary axis and the y-axis determines the horizontal rotation of the myocardium, ensuring alignment with the canonical position.

Re-oriented first model is then sliced in the step **314** into slices corresponding to the slices (cross-sections) of the second model obtained in the step **324.** Slices are taken in plane perpendicular to the longitudinal axis of the first model.

Then subsequent slices of the re-oriented first model are processed in step **331** and quality of segmentation thereof is checked in the step **332.** The analysis in the step **332** concerns one slice (cross-section) at a time starting with the one containing the myocardium's apex. The process is based on the presumption that myocardial cross-sections fall into one of two groups: 1) one or more simple polygons **212** located below or above the septum height as shown in Fig. 2c,d and 2) structures topologically equivalent to an annulus **211** in myocardium part along the septum height as shown in Fig. 2b. Group of slices classified as topologically equivalent (topological homeomorphism) to an annulus **211** should be contiguous. It is unexpected if a slice topologically equivalent to polygon **212** is detected in-between slices topologically equivalent to annulus **211.** If unexpected presence of one or more slices with a different classification in-between slices **211** is detected, it is considered as an error in segmentation with vision transformer. Slices with incorrect topology, that is the slices not having a topological homeomorphism to annulus but located between two slices having topological homeomorphism to the annulus are to be repaired.

In the step **333** decision is made. If there are no more slices in the model the iterative analysis is finished.

If the result of analysis in step **332** indicated correct topology next slice is taken. If the result of analysis in step **332** indicated wrong topology the slice is replaced with corresponding slice from the second model in step **335.**

It is noted that use of other ensembling method is also possible. In particular both models can be subjected to analysis and scoring of slices can be applied.

If the result of analysis in the step **332** indicated incorrect topology i.e. the topology of the slice is identified as incorrect, correction for the faulty slice is initiated. The faulty slice is replaced with a corresponding slice from the second model in the step **335.** Alternatively, areas of the slice of the first model and the slice of the second model are merged, thereby fixing the integrity of the resulting myocardial structure.

An efficient way to distinguish shapes **211** from shapes **212** is using Euler characteristic. Annulus **211** has Euler characteristic of "0", shapes **212** have Euler characteristics of "1" or higher. More complicated erroneous slices could have Euler characteristic below "0", representing multiple partitions.

Upon achieving a topologically correct volumetric model it is subjected to re-orientation in the step **341** as replacement of slices may change the shape of the model and repositioning of characteristic points.

Subsequently topologically correct volumetric model is transformed to mesh model in the step **342.** In the present embodiment marching cube algorithm is applied therefor.

In the further optional steps the mesh model is subjected to remeshing **343** and smoothing **344.** The remeshing step **343** rectifies various issues in the mesh e.g. duplicate faces, self-intersections, too short edges, degenerated triangles, and isolated vertices. Adequate smoothing technique for the step **344** is Taubin smoothing.

Result of ensembling step **133** is outputted in the step **345.**

The ensembled model is used in the step **160** of computational fluid dynamics simulation. The simulation may be done over larger part of vascular system and the ensemble model of myocardium or with sole ensembled model of myocardium but with parameters defining boundary conditions. This step comprises extraction **163** of at least one additional parameter.

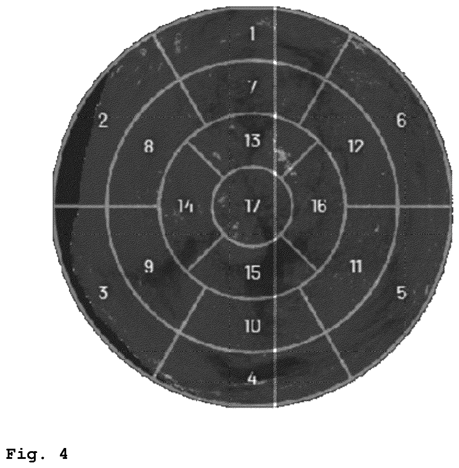

Said parameter can be used for adding color or shades into an image of myoacardium represented in AHA scale - see Fig. 4.

Alternatively, more than one additional parameter can determined in the step **160** of computational fluid dynamics and presented in graphical or numerical form. It has been tested, that particularly advantageous include myocardial blood flow (MBF), time to peak (TTP), tissue transit time (TTT) and myocardial blood volume (MBV). Further options CNBP - continuous blood pressure.

The ensembled model undergoes medical segmentation step 150 based on a preselected AHA cardiac scale. The system for segmenting the myocardium into 17 segments involves clustering the myocardium oriented in the canonical position by its height. The bottom-most 10% of the height is considered to be the 17th segment, and the remaining 90% of the height is divided equally into three groups. The bottom of those groups is divided into four segments, while the other two groups are divided into six segments each. The division of clusters is done with equal angular distances, a process facilitated by knowing the longitudinal axis and septal part center. The ensemble model and the 17 segments of the AHA classification are then visualized in step **170.**

The method according to the invention is computer implemented and can be realized by a personal computer server, a signal processor, implemented in dedicated system on chip device or in distributed architecture all of which are considered to be computers. The computer program product may be distributed by a network, stored on read only memory, or on a carrier. The program according to the invention comprises instructions which, when the program is executed by a computer, cause the computer to carry out the method according to the invention.

A client server architecture of computer system is particularly suited for use of the invention in the existing enterprises. The physician performing diagnosis operates terminal which is adapted to upload data to the server and display returned result. The server is adapted to process the data and return the result while the terminal is further adapted to visualize the result. The server is adapted to realize the method according to the invention and deliver the prepared to visualization to the terminal.

Visualization being result of the method according to the invention in the present embodiment is a combination of .stl file and .json file comprising orientation data, segmentation data and colormap representing additional parameter - MBF obtained in simulation. Such visualization is ready to be shown to the physician on the terminal.

Even though the embodiments described above refer to myocardium, it is noted that the method, computer program product and the system according to the invention are applicable also for other organs, especially when the model of the organ is to be both shown to the physician used for CFD simulations.

The computers, in the sense of the above description, are to be understood as hardware devices including computers, microcontrollers, signal processing, programmable gate arrays, understood as hardware computers, graphic cards, application-specific integrated circuits, or other processing digital devices used for image processing, as well as distributed solutions including cloud computing environments.

Those skilled in the art, given the teachings of the above description, are able to routinely propose multiple hardware and software solutions for device, according to the invention, and for computer-implemented execution of the method, according to the invention, also as methods for obtaining training information.

It is noted that described invention is applicable for diagnosis based on medical imaging of various organs - especially the ones that process fluids and may be subjected to CFD modeling.

In the claims, any reference signs placed between parentheses shall not be construed as limiting the claim. The use of the verb "comprise" does not mean that there are no elements or steps other than those stated in a claim. The article "a" or "an" preceding an element does not exclude the presence of a plurality of such elements. The invention may be implemented by means of hardware, comprising several distinct elements, and by means of a suitably programmed computer. In the device claim enumerating several means, several of these means may be embodied by one and the same item of hardware or separate items of hardware. The mere fact that certain measures are recited in mutually different, dependent claims, does not indicate that a combination of these measures cannot be used to advantage.

## Claims

1. A computer implemented method for enhancing diagnosis by processing medical images, comprising
a step **(110)** of receiving a medical image of an organ comprising three dimensional representation thereof,
a step **(120)** of preprocessing the medical image,
a step **(130)** of semantic segmentation of the medical image to obtain a model of the organ, and
a step **(140)** of orientation of the model,
a step **(150)** of medical segmentation of the model of the organ
a step **(170)** of preparing visualization of the model of the organ and/or the medical image together with the result of medical segmentation
**characterized in that**
the model of the organ is an ensembled model,
the step **(130)** of semantic segmentation comprises
applying a vision transformer **(131)** to obtain a first model of the organ,
applying a mesh deformer **(132)** to obtain a second model of the organ, and
ensembling **(133)** of the first model and the second model to the obtain ensembled model.

2. Computer implemented method according to claim 1, further comprising a step **(160)** of computational fluid dynamics simulation comprising
receiving patient specific parameters **(161)** for formulation boundary conditions,
running the computational fluid dynamics simulation with boundary conditions to determine **(163)** an additional parameter,
wherein the additional parameter is included in the visualization prepared in the step **(170).**

3. A computer implemented method according to claim 1 or 2, for enhancing diagnosis of cardiovascular diseases wherein
the organ is myocardium,
the step **(150)** of medical segmentation comprises cardiac segmentation according to a cardiac scale.

4. Computer implemented method according to claim 1 or 2 or 3, wherein medical image is a computed tomography angiography image.

5. Computer implemented method according to claim 3 or 4, wherein cardiac scale is 17 AHA scale.

6. Computer implemented method according to any of claims from 1 to 5, wherein the ensembling **(133)** comprises using a second model to repair the first model in case of detection of error.

7. Computer implemented method according to any of claims from 2 to 6, wherein the at additional parameter determined in the step **(160)** of computational fluid dynamics simulation is selected from a group comprising myocardial blood flow, time to peak, tissue transit time and myocardial blood volume.

8. Computer implemented method according to any of claims 2 to 7, wherein the step **(160)** of computational fluid dynamics simulation is used to determine two or more additional parameters which are then used in the step **(170)** of preparing visualization.

9. Computer implemented method according to any of claims 6 to 8, wherein the ensembling **(133)** comprises analysis of the first model obtained with vision transformer **(131)** in subsequent slices **(210),** wherein every slice is analyzed with respect to topological homeomorphism to a set of predefined structures and the slice that does not have expected topological homeomorphism is corrected with corresponding slice from the second model obtained with mesh deformer **(132).**

10. Computer implemented method according to any of claims 1 to 9, wherein the ensembling **(133)** comprises postprocessing of the ensambled model.

11. Computer program product comprising instructions which, when the program is executed by a computer, cause the computer to carry out the method of any of the claims 1 to 10.

12. A computer system comprising a terminal and a server wherein the terminal is adapted to upload data to the server and the server is adapted to process the data and return the result back to the terminal while the terminal is further adapted to visualize the result, **characterized in that** the server is adapted to realize the method as defined in any of the claims 1-9 and the terminal is adapted to show the visualization.

## Patentansprüche

1. Ein computerimplementiertes Verfahren zur Verbesserung einer Diagnose durch Verarbeitung medizinischer Bilder, umfassend
einen Schritt **(110)** des Empfangens eines medizinischen Bildes eines Organs, das eine dreidimensionale Darstellung desselben umfasst,
einen Schritt **(120)** des Vorverarbeitens des medizinischen Bildes,
einen Schritt **(130)** des semantischen Segmentierens des medizinischen Bildes, um ein Modell des Organs zu erhalten, und
einen Schritt **(140)** des Ausrichtens des Modells,
einen Schritt **(150)** des medizinischen Segmentierens des Modells des Organs
einen Schritt **(170)** des Vorbereitens der Visualisierung des Modells des Organs und/oder des medizinischen Bildes zusammen mit dem Ergebnis der medizinischen Segmentierung
**dadurch gekennzeichnet, dass**
das Modell des Organs ein zusammengesetztes Modell ist,
der Schritt **(130)** des semantischen Segmentierens umfasst
ein Anwenden eines Vision-Transformers **(131),** um ein erstes Modell des Organs zu erhalten,
ein Anwenden eines Netzdeformers **(132),** um ein zweites Modell des Organs zu erhalten, und
ein Zusammenfügen **(133)** des ersten Modells und des zweiten Modells, um ein zusammengesetztes Modell zu erhalten.

2. Computerimplementiertes Verfahren nach Anspruch 1, des Weiteren umfassend einen Schritt **(160)** einer nummerischen Strömungssimulation umfassend
ein Empfangen patientenspezifischer Parameter **(161)** zur Formulierung von Randbedingungen,
ein Ausführen der numerischen Strömungssimulation mit Randbedingungen, um einen zusätzlichen Parameter zu bestimmen **(163),**
wobei der zusätzliche Parameter in die in Schritt **(170)** vorbereitete Visualisierung einbezogen wird.

3. Computerimplementiertes Verfahren nach Anspruch 1 oder 2 zur Verbesserung einer Diagnose von Herz-Kreislauf-Erkrankungen, wobei
das Organ das Myokard ist,
der Schritt **(150)** des medizinischen Segmentierens eine, ein Herz betreffende Segmentierung gemäß einer kardialen Skala umfasst.

4. Computerimplementiertes Verfahren nach Anspruch 1 oder 2 oder 3, wobei das medizinische Bild ein berechnetes Tomographie-Angiographiebild ist.

5. Computerimplementiertes Verfahren nach Anspruch 3 oder 4, wobei die kardiale Skala eine 17-AHA-Skala ist.

6. Computerimplementiertes Verfahren nach einem der Ansprüche von 1 bis 5, wobei das Zusammenfügen **(133)** ein Verwenden eines zweiten Modells umfasst, um das erste Modell im Falle eines Erkennens eines Fehlers zu reparieren.

7. Computerimplementiertes Verfahren nach einem der Ansprüche von 2 bis 6, wobei der in Schritt **(160)** der numerischen Strömungssimulation bestimmte zusätzliche Parameter aus einer Gruppe ausgewählt wird, die einen myokardialen Blutfluss, eine Zeit bis zum Erreichen des Spitzenwerts, eine Gewebetransitzeit und ein myokardiales Blutvolumen umfasst.

8. Computerimplementiertes Verfahren nach einem der Ansprüche 2 bis 7, wobei der Schritt **(160)** der numerischen Strömungssimulation dazu verwendet wird, zwei oder mehr zusätzliche Parameter zu bestimmen, die dann im Schritt **(170)** der Vorbereitung der Visualisierung verwendet werden.

9. Computerimplementiertes Verfahren nach einem der Ansprüche 6 bis 8, wobei das Zusammenfügen **(133)** eine Analyse des mit dem Vision-Transformer **(131)** erhaltenen ersten Modells in aufeinanderfolgenden Schichten **(210)** umfasst, wobei jede Schicht hinsichtlich ihrer topologischen Homöomorphie zu einer Reihe vordefinierter Strukturen analysiert wird und eine Schicht, die nicht eine erwartete topologische Homöomorphie aufweist, mit der entsprechenden Schicht aus dem mit dem Netzdeformer **(132)** erhaltenen zweiten Modells korrigiert wird.

10. Computerimplementiertes Verfahren nach einem der Ansprüche 1 bis 9, wobei das Zusammenfügen **(133)** eine Nachbearbeitung des zusammengefügten Modells umfasst.

11. Computerprogrammprodukt, das Anweisungen umfasst, die, wenn das Programm von einem Computer ausgeführt wird, den Computer veranlassen, das Verfahren nach einem der Ansprüche 1 bis 10 auszuführen.

12. Computersystem, das ein Terminal und einen Server umfasst, wobei das Terminal dafür ausgelegt ist, Daten auf den Server hochzuladen, und der Server dafür ausgelegt ist, die Daten zu verarbeiten und das Ergebnis an das Terminal zurückzugeben, während das Terminal des Weiteren dafür ausgelegt ist, das Ergebnis zu visualisieren, **dadurch gekennzeichnet, dass** der Server dafür ausgelegt ist, das in einem der Ansprüche 1 bis 9 definierte Verfahren zu realisieren, und das Terminal dafür ausgelegt ist, die Visualisierung anzuzeigen.

## Revendications

1. Un procédé mis en œuvre par ordinateur pour améliorer le diagnostic en traitant des images médicales, comprenant
une étape **(110)** de réception d'une image médicale d'un organe comprenant sa représentation tridimensionnelle,
une étape **(120)** de prétraitement de l'image médicale,
une étape **(130)** de segmentation sémantique de l'image médicale pour obtenir un modèle de l'organe, et
une étape **(140)** d'orientation du modèle,
une étape **(150)** de segmentation médicale du modèle de l'organe
une étape **(170)** de préparation de la visualisation du modèle de l'organe et/ou de l'image médicale, accompagnée du résultat de la segmentation médicale
**caractérisé en ce**
Le modèle de l'organe est un modèle d'ensemble,
L'étape **(130)** de la segmentation sémantique comprend
L'application d'un transformateur de vision **(131)** pour obtenir un premier modèle de l'organe,
L'application d'un déformateur de maille **(132)** pour obtenir un second modèle de l'organe, et
L'assemblage **(133)** du premier modèle et du second modèle pour obtenir le modèle d'ensemble.

2. Procédé mis en œuvre par ordinateur selon la revendication 1, comprenant en outre une étape **(160)** de simulation numérique de dynamique des fluides comprenant
La réception des paramètres spécifiques au patient **(161)** pour formuler les conditions aux limites,
L'exécution de la simulation numérique de dynamique des fluides avec les conditions aux limites pour déterminer **(163)** un paramètre supplémentaire,
Dans lequel le paramètre supplémentaire est inclus dans la visualisation préparée à l'étape **(170).**

3. Un procédé mis en œuvre par ordinateur selon la revendication 1 ou 2, pour améliorer le diagnostic des maladies cardiovasculaires dans lesquelles
L'organe est le myocarde,
L'étape **(150)** de la segmentation médicale comprend la segmentation cardiaque selon une échelle cardiaque.

4. Procédé mis en œuvre par ordinateur selon la revendication 1, 2 ou 3, dans lequel l'image médicale est une image d'angiographie par tomodensitométrie.

5. Procédé mis en œuvre par ordinateur selon la revendication 3 ou 4, dans lequel l'échelle cardiaque est de 17 AHA.

6. Procédé mis en œuvre par ordinateur selon l'une quelconque des revendications de 1 à 5, dans lequel l'assemblage **(133)** consiste à utiliser un second modèle pour réparer le premier modèle en cas de détection d'erreur.

7. Procédé mis en œuvre par ordinateur selon l'une quelconque des revendications 2 à 6, dans lequel le paramètre supplémentaire déterminé à l'étape **(160)** de la simulation numérique de dynamique des fluides est sélectionné dans un groupe comprenant le flux sanguin myocardique, le temps jusqu'au pic, le temps de transit tissulaire et le volume sanguin myocardique.

8. Procédé mis en œuvre par ordinateur selon l'une quelconque des revendications 2 à 7, dans lequel l'étape **(160)** de simulation numérique de la dynamique des fluides est utilisée pour déterminer deux paramètres supplémentaires ou plus, qui sont ensuite utilisés à l'étape **(170)** de la préparation de la visualisation.

9. Procédé mis en œuvre par ordinateur selon l'une des revendications 6 à 8, dans lequel l'assemblage **(133)** comprend l'analyse du premier modèle obtenu avec le transformateur de vision **(131)** dans des tranches successives **(210),** dans lequel chaque tranche est analysée selon l'homéomorphisme topologique par rapport à un ensemble de structures prédéfinies et la tranche qui n'a pas d'homéomorphisme topologique attendu est corrigée avec la tranche correspondante du second modèle obtenu avec le déformateur de maille **(132).**

10. Procédé mis en œuvre par ordinateur selon l'une quelconque des revendications 1 à 9, dans lequel l'assemblage **(133)** comprend le post-traitement du modèle d'ensemble.

11. Produit programme d'ordinateur comprenant des instructions qui, lorsque le programme est exécuté par un ordinateur, font que l'ordinateur exécute le procédé selon l'une quelconque des revendications 1 à 10.

12. Un système informatique comprenant un terminal et un serveur dans lequel le terminal est adapté pour téléverser les données vers le serveur et le serveur est adapté pour traiter les données et renvoyer le résultat au terminal tandis que le terminal est en outre adapté pour visualiser le résultat, **caractérisé en ce que** le serveur est adapté pour mettre en oeuvre le procédé tel que défini dans l'une quelconque des revendications 1 à 9 et **en ce que** le terminal est adapté pour afficher la visualisation.
